(19) Europäisches Patentamt
European Patent Office
Office européen des brevets

(11) Veröffentlichungsnummer: **0 023 575**
**A1**

# (12) EUROPÄISCHE PATENTANMELDUNG

(21) Anmeldenummer: **80103750.8**

(22) Anmeldetag: **02.07.80**

(51) Int. Cl.³: **C 07 D 209/46**, C 07 D 217/24, C 07 D 401/12, C 07 D 403/12, A 61 K 31/395

(30) Priorität: **05.07.79 DE 2927129**

(43) Veröffentlichungstag der Anmeldung: **11.02.81 Patentblatt 81/6**

(84) Benannte Vertragsstaaten: **AT BE CH DE FR GB IT LI NL**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Weyer, Rudi, Dr., Johann-Strauss-Strasse 43, D-6233 Kelkheim (Taunus) (DE)**
Erfinder: **Hitzel, Volker, Dr., Kantstrasse 1b, D-6238 Hofheim am Taunus (DE)**
Erfinder: **Geisen, Karl, Dr., Jahnstrasse 43, D-6000 Frankfurt am Main 71 (DE)**
Erfinder: **Regitz, Günter, Dr., Dachbergstrasse 68, D-6232 Bad Soden am Taunus (DE)**

(54) **Benzolsulfonylsemicarbazide, deren Herstellung und Verwendung, sie enthaltende Arzneimittel und deren Herstellung.**

(57) Sulfonylsemicarbazide der Formel

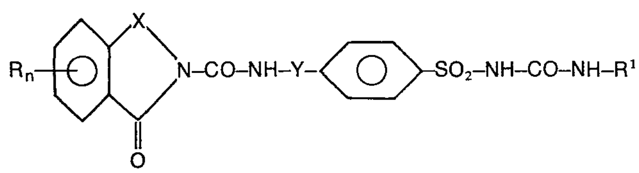

worin R, R¹, X, Y und n die angegebenen Bedeutungen haben, deren physiologisch verträgliche Salze, pharmazeutische Präparate auf Basis dieser Verbindungen sowie ihre Verwendung bei der Behandlung von Diabetes.

EP 0 023 575 A1

HOECHST AKTIENGESELLSCHAFT HOE 79/F 172 Dr.D/cr

# Sulfonylsemicarbazide, Verfahren zu ihrer Herstellung, pharmazeutische Präparate auf Basis dieser Verbindungen und ihre Verwendung

Die Erfindung betrifft Sulfonylsemicarbazide der Formel

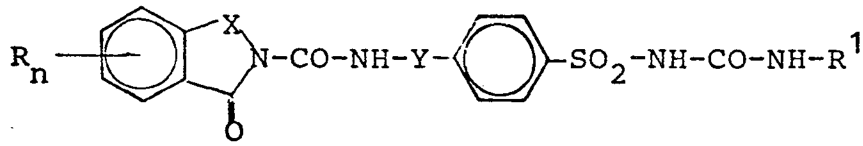

die als Substanz oder in Form ihrer physiologisch verträglichen Salze blutzuckersenkende Eigenschaften besitzen und sich durch starke und langanhaltende Senkung des Blutzuckerspiegels auszeichnen und daher als Arzneimittel verwendet werden können.

In der Formel bedeuten:

n 1 oder 2,

R Wasserstoff, Alkyl mit 1 bis 4 Kohlenstoffatomen, Alkoxy mit 1 bis 4 Kohlenstoffatomen oder Halogen, wobei falls n 2 ist,die R gleich oder verschieden sein können

X $-CH_2-$, $-CH_2-CH_2-$ oder $\overset{CH_3}{\overset{|}{-CH-}}$

Y Alkylen mit 2-3 C-Atomen

$R^1$ Alkyleniminorest mit 4-7 C-Atomen im Ring, der gegebenenfalls durch ein oder 2 Methylgruppen oder eine Äthylgruppe substituiert ist, oder ab einer Ringgröße mit 5 C-Atomen durch eine Endoalkylengruppe mit 1 bis 2 C-Atomen oder bei einer Ringgröße mit 7 C-Atomen durch eine Bindung zu einem bicyclischen System abgewandelt ist.

BAD ORIGINAL

In der allgemeinen Formel bedeutet R vorzugsweise Wasserstoff, Methyl und Halogen, insbesondere Chlor. Im Falle der Disubstitution sind die Dichlorverbindungen bevorzugt. Besonders bevorzugt ist für R Wasserstoff. X bedeutet vorzugsweise die $-CH_2$-Gruppe und Y vorzugsweise $-CH_2-CH_2-$, $-\underset{\displaystyle CH_3}{\underset{|}{CH}}-CH_2-$, wobei die $-CH_2-CH_2$-Gruppe besonders bevorzugt ist. $R^1$ ist vorzugsweise der Pentamethyleniminorest oder der Hexamethyleniminorest. Die Endoalkylengruppe hat vorzugsweise 1 C-Atom.

Die Erfindung betrifft ferner Verfahren zur Herstellung dieser Sulfonylsemicarbazide, pharmazeutische Präparate, die diese enthalten oder aus ihnen bestehen sowie die Verwendung zur Behandlung des Diabetes.

Die Verfahren zur Herstellung sind dadurch gekennzeichnet, daß man

a) mit der Gruppe

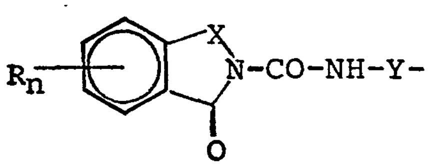

in 4-Stellung substituierte Benzolsulfonyl-isocyanate, -carbaminsäureester, -thiolcarbaminsäureester, oder -harnstoffe, mit einem Amin $R^1-NH_2$ oder dessen Salzen umsetzt oder Sulfonamide der Formel

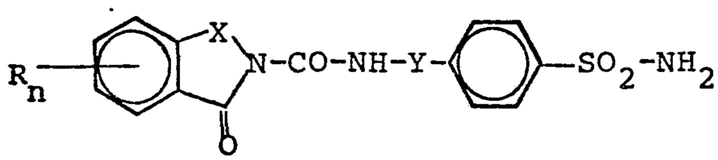

oder deren Salze mit $R^1$-substituierten Carbaminsäureestern, Thiolcarbaminsäureestern, oder Harnstoffen umsetzt,

b) mit der Gruppe

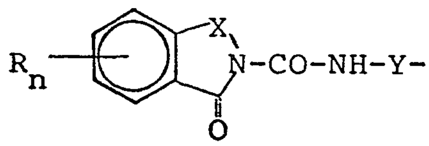

substituierte Benzolsulfonyl-isosemicarbazidäther, -isothiosemicarbazidäther, oder -parabansäuren spaltet,

c) in

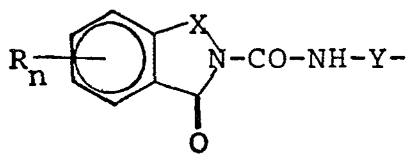

substituierten Benzolsulfonylthiosemicarbaziden das Schwefelatom durch Sauerstoff ersetzt,

d) in Benzolsulfonylsemicarbazide der Formel

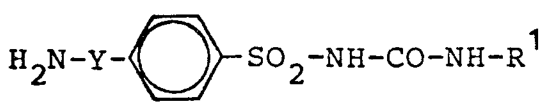

gegebenenfalls stufenweise den Rest

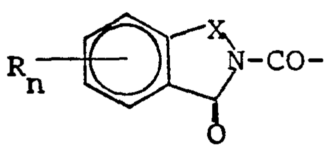

einführt,

e) entsprechend substituierte Benzolsulfonylhalogenide mit $R^1$-substituierten Harnstoffen oder deren Alkalisalzen umsetzt und die Reaktionsprodukte gegebenenfalls zur Salzbildung mit alkalischen Mitteln oder physiologisch verträglichen anorganischen oder organischen Säuren behandelt.

Die erwähnten Benzolsulfonyl-carbaminsäureester bzw. -thiolcarbaminsäureester können in der Alkoholkomponente einen Alkylrest oder einen Arylrest oder auch einen heterocyclischen Rest aufweisen. Da dieser Rest bei der Reaktion abgespalten wird, hat seine chemische Konstitution keinen Einfluß auf den Charakter des Endproduktes und kann deshalb in weiten Grenzen variiert werden. Das gleiche gilt für die N-$R^1$-substituierten Carbaminsäureester bzw. die entsprechenden Thiolcarbaminsäureester.

Die als Ausgangsstoffe des Verfahrens infrage kommenden Benzolsulfonylharnstoffe können an der der Sulfonylgruppe abgewandten Seite des Harnstoffmoleküls unsubstituiert oder ein- oder insbesondere zweifach substituiert sein. Da diese Substituenten bei der Reaktion mit Aminen abgespalten werden, kann ihr Charakter in weiten Grenzen variiert werden. Neben alkyl-, aryl-, acyl- oder heterocyclisch substituierten Benzolsulfonylharnstoffen kann man auch Benzolsulfonylcarbamoylimidazole und ähnliche Verbindungen oder Bisbenzolsulfonylharnstoffe, die an einem der Stickstoffatome noch einen weiteren Substituenten z.B.Methyl, tragen können, verwenden. Man kann beispielsweise derartige Bis-(benzolsulfonyl)-harnstoffe oder auch N-Benzolsulfonyl-N'-acylharnstoffe mit $R^1$-substituierten Aminen behandeln und die erhaltenen Salze auf erhöhte Temperaturen, insbesondere solche oberhalb 100°C, erhitzen.

Weiterhin ist es möglich, von $R^1$-substituierten Harnstoffen auszugehen oder von solchen $R^1$-substituierten Harnstoffen, die am freien Stickstoffatom noch ein- oder insbesondere zweifach substituiert sind und diese mit

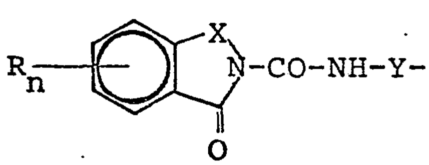

in 4-Stellung substituierten Benzolsulfonamiden umzusetzen. Als solche Harnstoffe eignen sich die der allg. Formel $R^1-NH-CO-NH_2$ oder acylierte Verbindungen der allgemeinen Formel $R^1$-NH-CO-NH-acyl, worin acyl einen vorzugsweise niedrigmolekularen aliphatischen oder aromatischen Säurerest bedeutet, oder Diphenylharnstoffe der Formel $R^1-NH-CO-N(C_6H_5)_2$, wobei die Phenylreste substituiert sowie auch direkt oder über eine Brücke wie $-CH_2-$, -NH-, -O-, oder -S- miteinander verbunden sein können oder N,N'-di-substituierte Carbohydrazine der Formel $R^1-NH-CO-NH-R^1$.

Die Spaltung der als Ausgangsstoffe in Verfahren b) genannten Benzolsulfonyliminoparabansäuren, -isosemicarbazidäther oder -isothiosemicarbazidäther erfolgt zweckmäßig durch alkalische Hydrolyse. Isosemicarbazidäther können auch in einem sauren Medium mit gutem Erfolg gespalten werden.

Der Ersatz des Schwefelatoms in der Semicarbazidgruppierung von entsprechend substituierten Benzolsulfonylthiosemicarbaziden durch ein Sauerstoffatom kann in bekannter Weise zum Beispiel mit Hilfe von Oxyden oder Salzen von Schwermetallen oder auch durch Anwendung von Oxydationsmitteln, wie Wasserstoffperoxid oder Natriumperoxid ausgeführt werden.

Die Acylierung der Sulfonylsemicarbazide gemäß Verfahren d) kann mit reaktiven Derivaten der Säure

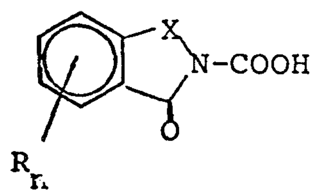

wie beispielsweise Halogeniden, erfolgen.
Als Benzolsulfonylhalogenide gemäß Verfahren e) eignen sich insbesondere die Chloride.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen und anorganische Säuren.

Die Ausführungsformen des Verfahrens gemäß der Erfindung können im allgemeinen hinsichtlich der Reaktionsbedingungen weitgehend variiert und den jeweiligen Verhältnissen angepaßt werden. Beispielsweise können die Umsetzungen in Abwesenheit oder Anwesenheit von Lösungsmitteln, bei Raumtemperatur oder bei erhöhter Temperatur durchgeführt werden.

Je nach dem Charakter der Ausgangsstoffe kann das eine oder andere der beschriebenen Verfahren in einzelnen Fällen ein gewünschtes individuelles Benzolsulfonylsemicarbazid nur in geringen Ausbeuten liefern oder zu dessen Synthese nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die erhaltenen Verbindungen können durch Umfällen und/oder Umkristallisieren gereinigt werden. Die Reinigung kann auch erfolgen, indem man die Substanz aus einem kristallinen (Alkali)-Salz in einem geeigneten Lösungsmittel in Freiheit setzt.

Die erfindungsgemäßen Verbindungen zeichnen sich durch

wertvolle pharmakologische Eigenschaften, insbesondere blutzuckersenkende, aus. Sie eignen sich daher als Arzneimittel, insbesondere als Antidiabetika.

Die blutzuckersenkende Wirkung der beschriebenen Benzolsulfonylsemicarbazide kann dadurch festgestellt werden, daß man sie als freie Verbindung oder in Form der Natriumsalze an normal ernährte Kaninchen verfüttert und den Blutzuckerwert nach der bekannten Methode von Hagedorn-Jensen oder mit einem Autoanalyzer über eine längere Zeitdauer ermittelt.

Die routinemäßige Bestimmung der blutzuckersenkenden Wirkung kann mit Dosierungen von z.B. 10,2 oder 0,4 mg Wirksubstanz pro kg Versuchstier nach bekannten Methoden erfolgen.

4-(4-<2-(1-Oxo-isoindolin-2-carboxamido)-äthyl>-benzolsulfonyl)-1,1-hexamethylen-semicarbazid wurde in einer Dosierung von 0,4 mg/kg Kaninchen verabreicht und die Blutzuckerwerte wurden mit einem Autoanalyzer über eine längere Zeitdauer ermittelt. Die hierbei gemessene Blutzuckersenkung ist in der nachfolgenden Tabelle in % nach ..... Stunden angegeben. In der Tabelle ist ferner die Blutzuckersenkung in % angegeben, die nach p.o. Verabreichung von 0,08 mg/kg Kaninchen beobachtet wurde.

Tabelle

Blutzuckersenkung an Kaninchen in % nach Verabreichung von mg/kg p.o. nach ... Stunden

| mg/kg | 1 | 3 | 5 | 24 | 48 |
|---|---|---|---|---|---|
| 0,4 | 26 | 31 | 43 | 37 | 0 |
| 0,08 | 11 | 22 | 33 | | |

Die erfindungsgemäßen Acylureidoalkylbenzolsulfonylsemicarbazide zeichnen sich durch eine starke und langanhaltende blutzuckersenkende Wirkung aus. Außerdem sind die Verbindungen gut verträglich.

Die Eigenschaften der Verbindungen erlauben es, in der Therapie des Diabetes mellitus mit so geringen Dosen auszukommen, daß das Präparat nur die verminderte Ansprechbarkeit des Pankreas auf einen erhöhten Blutzuckerspiegel wieder normalisiert.

Benzolsulfonylharnstoffe mit einem Ureidoalkylrest sind schon mehrfach beschrieben worden (DE-PS 14 43 911, DE-AS 16 70 700, DE-PS 16 18 389, DE-AS 22 38 870). Benzolsulfonylsemicarbazide mit einem Acylureidoalkylrest sind noch nicht bekannt und es war nicht zu erwarten, daß sie sich durch die oben erwähnten günstigen Eigenschaften auszeichnen.

Die beschriebenen Sulfonylsemicarbazide sollen vorzugsweise zur Herstellung von oral verabreichbaren Präparaten zur Behandlung des Diabetes mellitus dienen. So können als solche oder in Form ihrer Salze bzw. in Gegenwart von Stoffen, die zu einer Salzbildung führen, appliziert werden. Zur Salzbildung können beispielsweise alkalische Mittel wie Alkali- oder Erdalkalihydroxyde, -carbonate oder -bicarbonate herangezogen werden. Die Präparate können neben den Sulfonylsemicarbaziden bzw. dessen Salz auch noch andere Wirkstoffe enthalten.

Als medizinische Präparate kommen vorzugsweise Tabletten in Betracht, die neben den Verfahrenserzeugnissen die üblichen Träger- und Hilfsstoffe wie Talkum, Stärke, Milchzucker oder Magnesiumstearat enthalten. Dabei kann es zweckmäßig sein, den oder die Wirkstoffe in gemahlener oder fein gefällter Form oder als Gemisch dieser Formen einzusetzen. Ein Präparat, das die beschriebenen Benzolsulfonylsemicarbazide als Wirkstoff enthält, z.B. eine Tablette oder ein Pulver mit oder ohne Zusätze, ist zweckmäßig in eine geeignet dosierte Form gebracht. Als Dosis ist dabei eine solche zu wählen, die der Wirksamkeit des verwendeten Benzolsulfonylsemicarbazids und dem gewünschten Effekt angepaßt ist. Zweckmäßig beträgt die Dosierung je Einheit etwa 5 bis 50 mg, vorzugsweise 2 bis 10mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemäßen Sulfonylsemicarbazide verwendet werden können. Sie sollen jedoch nicht eine Einschränkung des Erfindungsgegenstandes darstellen.

Beispiel 1:

4-(4-⟨2-(1-Oxo-isoindolin-2-carboxamido)-äthyl⟩-benzolsulfonyl)-1,1-pentamethylen-semicarbazid

4,2 g N-(4-⟨2-(1-Oxo-isoindolin-2-carboxamido)-äthyl⟩-benzolsulfonyl)-carbamidsäuremethylester (hergestellt durch Umsetzung von 1-Oxo-isoindolin mit 2-Phenyläthylisocyanat zum 1-Oxo-isoindolin-2-(N-2-phenyläthyl)-carboxamid vom Schmp. 146 - 148°, Umsetzung dieser Verbindung mit Chlorsulfonsäure zum Sulfochlorid, Reaktion des Sulfochlorids mit Ammoniak zum Sulfonamid vom Schmp. 236 - 238° und Umsetzung des Sulfonamids mit Chlorameisensäuremethylester zum N-(4-⟨2-(1-Oxo-isoindolin-2-carboxamido)-äthyl⟩-benzolsulfonyl)-carbamidsäuremethylester vom Schmp. 215 - 217°) werden mit 1,5 g N-Amino-piperidin in
100 ml Dioxan
1 1/2 Stunden am Rückflußkühler gekocht.
Anschließend engt man die Lösung unter vermindertem Druck ein, versetzt den Rückstand mit verdünnter Essigsäure, behandelt die abgeschiedene zähe Substanz mit sehr verdünntem Ammoniak, filtriert und säuert wieder mit Essigsäure an. Das ausgefällte 4-(4-⟨2-(1-Oxo-isoindolin-2-carboxamido)-äthyl⟩-benzolsulfonyl)-1,1-pentamethylen-semicarbazid wird aus Methanol-Dioxan umkristallisiert und schmilzt bei 216 - 218°.
In analoger Weise erhält man das
4-(4-⟨2-(1-Oxo-isoindolin-2-carboxamido)-äthyl⟩-benzolsulfonyl)-1,1-hexamethylen-semicarbazid
vom Schmp. 210 - 212° (aus Methanol-Dioxan)

4-(4-⟨2-(1-Oxo-isoindolin-2-carboxamido)-äthyl⟩-benzolsulfonyl)-1,1-(3-methyl-pentamethylen)-semicarbazid
vom Schmp. 222 - 224° (aus Methanol-Dioxan)

Beispiel 2:

4-(4-⟨2-(5-Chlor-1-oxo-isoindolin-2-carboxamido)-äthyl⟩-benzolsulfonyl)-1,1-pentamethylen-semicarbazid

2,3 g N-(4-⟨2-(5-Chlor-1-oxo-isoindolin-2-carboxamido)-äthyl⟩-benzolsulfonyl)-carbamidsäure-methylester (hergestellt durch Umsetzung von 5-Chlor-phthalimidin mit 2-Phenyl-äthylisocyanat zum 5-Chlor-1-oxo-isoindolin-2-(N-2-phenyläthyl)-carboxamid vom Schmp. 160 - 162°, Umsetzung dieser Verbindung mit Chlorsulfonsäure zum Sulfochlorid, Reaktion des Sulfochlorids mit Ammoniak zum Sulfonamid vom Schmp. 245 - 247° und Umsetzung des Sulfonamids mit Chlorameisensäuremethylester zum N-(4-⟨2-(5-Chlor-1-oxo-isoindolin-2-carboxamido)-äthyl⟩-benzolsulfonyl)-carbamidsäuremethylester vom Schmp. 220 - 222°) werden mit
0,75 g N-Amino-piperidin in
100 ml Dioxan
1 1/2 St. unter Rückfluß zum Sieden erhitzt.
Anschließend engt man die Lösung unter vermindertem Druck ein, versetzt den Rückstand mit verdünnter Essigsäure, behandelt die nicht kristalline Fällung mit sehr verdünntem Ammoniak, filtriert und säuert das Filtrat mit verdünnter Essigsäure an. Das ausgefällte 4-(4-⟨2-(5-Chlor-1-oxo-isoindolin-2-carboxamido)-äthyl⟩-benzolsulfonyl)-1,1-pentamethylen-semicarbazid wird aus Methanol-Dioxan umkristallisiert und schmilzt bei 215 - 217°.

**Beispiel 3:**

**4-(4-⟨2-(1-Oxo-isoindolin-2-carboxamido)-propyl⟩-benzolsulfonyl)-1,1-pentamethylen-semicarbazid**

4,3 g N-(4-⟨2-(1-Oxo-isoindolin-2-carboxamido)-propyl⟩-benzolsulfonyl)-carbamidsäuremethylester (hergestellt durch Umsetzung von 1-Oxo-isoindolin mit 2-Phenylpropyl-isocyanat zum 1-Oxo-isoindolin-2-(N-2-phenylpropyl)-carboxamid vom Schmp. 126 - 128°, Umsetzung dieser Verbindung mit Chlorsulfonsäure zum Sulfochlorid, Reaktion des Sulfochlorids mit Ammoniak zum Sulfonamid vom Schmp. 183 - 185° und Umsetzung des Sulfonamid mit Chlorameisensäuremethylester zum N-(4-⟨2-(1-Oxo-isoindolin-2-carboxamido)-propyl⟩-benzolsulfonyl)-carbamidsäuremethylester vom Schmp. 195 - 197°)
werden mit
1 g N-Amino-piperidin in
150 ml Dioxan
1 Stunde am Rückflußkühler zum Sieden erhitzt.
Anschließend dampft man das Dioxan unter vermindertem Druck ab, behandelt den Rückstand mit sehr verdünntem Ammoniak, filtriert und säuert mit verdünnter Essigsäure an. Das ausgefällte 4-(4-⟨2-(1-Oxo-isoindolin-2-carbox-amido)-propy⟩-benzolsulfonyl)-1,1-pentamethylen-semicarbazid wird aus verd. Äthanol umkristallisiert und schmilzt bei 178 - 180°.

Beispiel 4:

4-(4-⟨2-(1-Oxo-1,2,3,4-tetrahydroisochinolin-2-carboxamido)-äthyl⟩-benzolsulfonyl)-1,1-pentamethylen-semicarbazid

4,3 g N-(4-⟨2-(1-Oxo-1,2,3,4-tetrahydro-isochinolin-2-carboxamido)-äthyl⟩-benzolsulfonyl)-carbamidsäuremethylester (hergestellt durch Umsetzung von 1-Oxo-1,2,3,4-tetrahydro-isochinolin mit 2-Phenyläthylisocyanat zum 1-Oxo-1,2,3,4-tetrahydro-isochinolin-2-(N-2-phenyläthyl)-carboxamid vom Schmp. 98 - 99°C, Umsetzung des Amids mit Chlorsulfonsäure zum Sulfochlorid, Reaktion des Sulfochlorids mit Ammoniak zum Sulfonamid vom Schmp. 197 - 198°C und Umsetzung des Sulfonamids mit Chlorameisensäuremethylester zum N-(4-⟨2-(1-Oxo-1,2,3,4-tetrahydro-isochinolin-2-carboxamido)-äthyl⟩-benzolsulfonyl)-carbamidsäuremethylester vom Schmp. 191 - 193°C) werden mit 1,1 g N-Amino-piperidin in 25 ml Dioxan 4 Stunden unter Rückfluß gerührt. Nach dem Erkalten gießt man auf Eis und säuert mit 2 N Salzsäure an. Das so erhaltene 4-(4-⟨2-(1-Oxo-1,2,3,4-tetrahydroisochinolin-2-carboxamido)-äthyl⟩-benzolsulfonyl)-1,1-pentamethylen-semicarbazid wird aus Äthanol umkristallisiert und schmilzt bei 174 - 175°C.

In analoger Weise erhält man das 4-(4-⟨2-(1-Oxo-1,2,3,4-tetrahydroisochinolin-2-carboxamido)-äthyl⟩-benzolsulfonyl)-1,1-hexamethylen -semicarbazid vom Schmp. 183 - 185°C (aus Äthanol)

Beispiel 5:

4-(4-⟨2-(1-Oxo-1,2,3,4-tetrahydroisochinolin-2-carboxamido)-äthyl⟩-benzolsulfonyl)-1,1-(1-methyl-pentamethylen)-semicarbazid

2,0 g N-(4-⟨2-(1-Oxo-1,2,3,4-tetrahydroisochinolin-2-carboxamido)-äthyl⟩-benzolsulfonyl)-harnstoff (Schmp. 200-201°C; hergestellt aus dem in Beispiel 4 beschriebenen Sulfonamid mit Kaliumcyanat) werden mit 1,2 g N-Amino-2-methyl-piperidin in 25 ml Dioxan 90 Minuten unter Rühren zum Rückfluß gebracht. Anschließend wird das Lösungsmittel im Vakuum entfernt, der Rückstand in Wasser gelöst und die Lösung nach dem Filtrieren mit 2N Salzsäure angesäuert. Nach nochmaligem Umfällen aus verd. Ammoniak-Lösung) verd. Salzsäure wird das 4-(4-⟨2-(1-Oxo-1,2,3,4-tetrahydroisochinolin-2-carboxamido)-äthyl⟩-benzolsulfonyl)-1,1-(1-methyl-pentamethylen)-semicarbazid aus Äthanol umkristallisiert und schmilzt bei 164 - 165°C.

In analoger Weise erhält man das 4-(4-⟨2-(1-Oxo-1,2,3,4-tetrahydroisochinolin-2-carboxamido)-äthyl⟩-benzolsulfonyl)-1,1-(3-methyl-pentamethylen)-semicarbazid vom Schmp. 198 - 200°C (aus Äthanol)

PATENTANSPRÜCHE

1. Sulfonylsemicarbazide der Formel

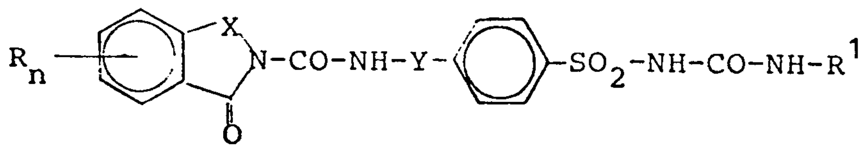

in welcher bedeuten:

n 1 oder 2,

R Wasserstoff, Alkyl mit 1 bis 4 Kohlenstoffatomen, Alkoxy mit 1 bis 4 Kohlenstoffatomen oder Halogen, wobei falls n 2 ist, die R gleich oder verschieden sein können

X $-CH_2-$, $-CH_2-CH_2-$ oder 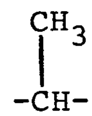

Y Alkylen mit 2-3 C-Atomen

$R^1$ Alkyleniminorest mit 4-7 C-Atomen im Ring, der gegebenenfalls durch ein oder 2 Methylgruppen oder eine Äthylgruppe substituiert ist, oder ab einer Ringgröße mit 5 C-Atomen durch eine Endoalkylengruppe mit 1 bis 2 C-Atomen oder bei einer Ringgröße mit 7 C-Atomen durch eine Bindung zu einem bicyclischen System abgewandelt ist,

und deren physiologisch verträgliche Salze.

2. Verfahren zur Herstellung von Sulfonylsemicarbaziden gemäß Anspruch 1, dadurch gekennzeichnet, daß man

a) mit der Gruppe

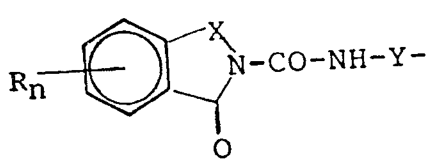

in 4-Stellung substituierte Benzolsulfonyl-isocyanate, -carbaminsäureester, -thiolcarbaminsäureester, oder -harnstoffe, mit einem Amin $R^1$-$NH_2$ oder dessen Salzen umsetzt oder Sulfonamide der Formel

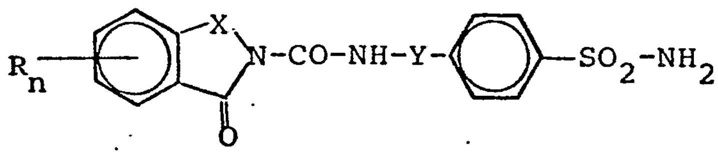

oder deren Salze mit $R^1$-substituierten Carbaminsäureestern, Thiolcarbaminsäureestern, oder Harnstoffen umsetzt,

b) mit der Gruppe

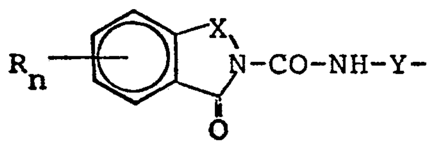

substituierte Benzolsulfonyl-isosemicarbazidäther, -isothiosemicarbazidäther, oder -parabansäuren spaltet,

c) in

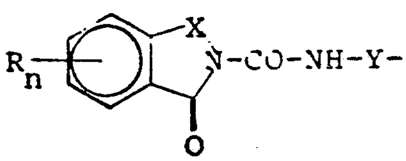

substituierten Benzolsulfonylthiosemicarbaziden das Schwefelatom durch Sauerstoff ersetzt,

d) in Benzolsulfonylsemicarbaziden der Formel

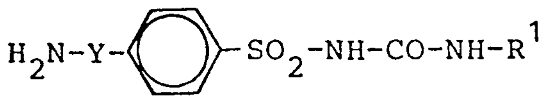

gegebenenfalls stufenweise den Rest

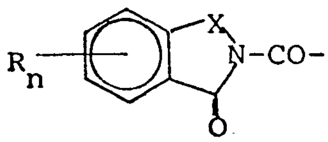

einführt,

e) entsprechend substituierte Benzolsulfonylhalogenide mit $R^1$-substituierten Harnstoffen oder deren Alkalisalzen umsetzt und die Reaktionsprodukte gegebenenfalls zur Salzbildung mit alkalischen Mitteln oder physiologisch verträglichen anorganischen oder organischen Säuren behandelt.

3. Arzneimittel enthaltend ein Sulfonylsemicarbazid gemäß Anspruch 1 oder eines seiner Salze.

4. Verwendung eines Sulfonylsemicarbazids gemäß Anspruch 1 oder eines seiner Salze bei der Bekämpfung von Diabetes.

5. Verfahren zur Herstellung eines Arzneimittels gemäß Anspruch 3, dadurch gekennzeichnet, daß man ein Sulfonylsemicarbazid der im Anspruch 1 angegebenen Formel oder eines seiner Salze in eine geeignete Applikationsform bringt.

Patentanspruch für Österreich:

Verfahren zur Herstellung von Sulfonylsemicarbaziden der Formel

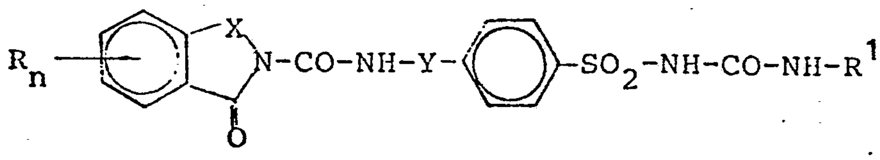

in welcher bedeuten:

n 1 oder 2,

R Wasserstoff, Alkyl mit 1 bis 4 Kohlenstoffatomen, Alkoxy mit 1 bis 4 Kohlenstoffatomen oder Halogen, wobei falls n 2 ist, die R gleich oder verschieden sein können

X $-CH_2-$, $-CH_2-CH_2-$ oder 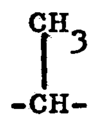

Y Alkylen mit 2-3 C-Atomen

$R^1$ Alkyleniminorest mit 4-7 C-Atomen im Ring, der gegebenenfalls durch ein oder 2 Methylgruppen oder eine Äthylgruppe substituiert ist, oder ab einer Ringgröße mit 5 C-Atomen durch eine Endoalkylengruppe mit 1 bis 2 C-Atomen oder bei einer Ringgröße mit 7 C-Atomen durch eine Bindung zu einem bicyclischen System abgewandelt ist,

und von deren physiologisch verträglichen Salzen, dadurch gekennzeichnet, daß man

a) mit der Gruppe

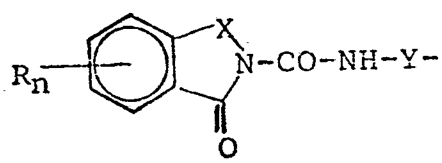

in 4-Stellung substituierte Benzolsulfonyl-isocyanate, -carbaminsäureester, -thiolcarbaminsäureester, oder -harnstoffe, mit einem Amin $R^1-NH_2$ oder dessen Salzen umsetzt oder Sulfonamide der Formel

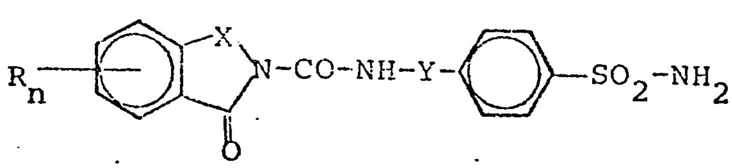

oder deren Salze mit $R^1$-substituierten Carbaminsäureestern, Thiolcarbaminsäureestern, oder Harnstoffen umsetzt,

b) mit der Gruppe

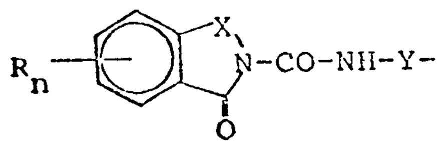

substituierte Benzolsulfonyl-isosemicarbazidäther, -isothiosemicarbazidäther, oder -parabansäuren spaltet,

c) in

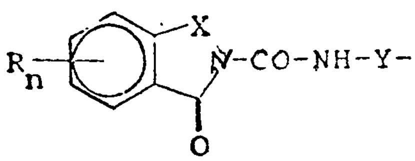

substituierten Benzolsulfonylthiosemicarbaziden das Schwefelatom durch Sauerstoff ersetzt,

d) in Benzolsulfonylsemicarbaziden der Formel

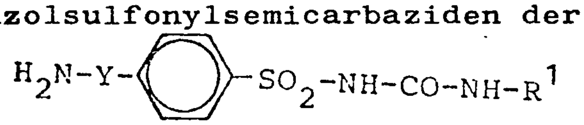

gegebenenfalls stufenweise den Rest

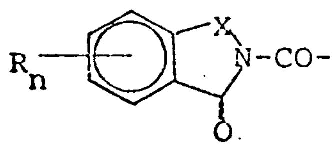

einführt,

e) entsprechend substituierte Benzolsulfonylhalogenide mit $R^1$-substituierten Harnstoffen oder deren Alkalisalzen umsetzt

und die Reaktionsprodukte gegebenenfalls zur Salzbildung mit alkalischen Mitteln oder physiologisch verträglichen anorganischen oder organischen Säuren behandelt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0023575

Nummer der Anmeldung
EP 80103750.8

**EINSCHLÄGIGE DOKUMENTE**

| Kategorie | Kennzeichnung des Dokuments mit Angabe, soweit erforderlich, der maßgeblichen Teile | betrifft Anspruch |
|---|---|---|
| | AT - B - 281 059 (BOEHRINGER)<br>+ Patentanspruch +<br>-- | 1-3 |
| | DE - A - 2 145 559 (NOVO TERAPEUTISK)<br>+ Seiten 1-4, 7-9 +<br>---- | 1-3 |

KLASSIFIKATION DER ANMELDUNG (Int. Cl.3)

C 07 D 209/46
C 07 D 217/24
C 07 D 401/12
C 07 D 403/12
A 61 K 31/395

RECHERCHIERTE SACHGEBIETE (Int. Cl.3)

A 61 K 31/00
C 07 C 133/00
C 07 C 143/00
C 07 D 217/00
C 07 D 209/00
C 07 D 401/00
C 07 D 403/00
C 07 D 295/00
C 07 D 307/00

**UNVOLLSTÄNDIGE RECHERCHE**

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5
Unvollständig recherchierte Patentansprüche:
Nicht recherchierte Patentansprüche: 4 (Verfahren zur therapeutischen Behandlung
Grund für die Beschränkung der Recherche: des menschlichen oder tierischen Körpers Art. 52(4) EPÜ)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 15-09-1980 | TENGLER |